# EUROPEAN PATENT APPLICATION

(11) **EP 4 471 795 A1**
(43) Date of publication of application: **04.12.2024**
(21) Application number: 23176948.0
(22) Date of filing: 02.06.2023
(51) Int. Cl.: G16H 40/63, A61B 5/00, A61M 16/00, G16H 50/30

(54) **SYSTEM AND METHOD FOR GENERATING PREDICTIVE INDICATOR OF A DIFFERENCE BETWEEN PAP TITRATION AND CONVENTIONAL SLEEP SESSIONS**

(71) Applicant: Koninklijke Philips N.V., 5656 AG Eindhoven (NL)
(72) Inventor: VAN ZANTEN, Joyce, Eindhoven (NL); DOS SANTOS DA FONSECA, Pedro Miguel Ferreira, Eindhoven (NL); VAN DEN ENDE, Daan Anton, 5656AG Eindhoven (NL); HENDRIKS, Cornelis Petrus, Eindhoven (NL); KAHLERT, Joachim Johannes, Eindhoven (NL)
(74) Representative: Philips Intellectual Property & Standards

(57) **Abstract**

A system and method for generating a predictive indicator representing a difference between a PAP titration sleep session and at least one conventional sleep session. Data relating to the subject's sleep and to sleep influencing factors is obtained for both the PAP titration sleep session and the conventional sleep session, and is processed to identify any differences between the PAP titration sleep session and the conventional sleep session. A predictive indicator indicative of whether the identified difference(s), if present, satisfy a first predetermined condition is generated.

## Description

### FIELD OF THE INVENTION

The invention relates to the field of positive airway pressure (PAP) therapy.

### BACKGROUND OF THE INVENTION

Sleep-disordered breathing (SDB) conditions, such as OSA and CSA, are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

PAP titrations often take place in clinical settings, such as sleep laboratories, hospitals or specially-adapted hotel rooms. In some cases, a PAP titration is carried out during the second half of a sleep study night, with a SDB condition having been diagnosed from a sleep study during the first half of the night. On the day of the PAP titration, the subject is required to conform to certain requirements (e.g. avoiding napping, not drinking alcohol or caffeine, avoiding the use of hair products that may interfere with the sleep recording, and not taking certain medication). Further, spending the night in a clinical setting naturally alters some aspects of the subject's behavior: for example, the subject will eat the meal provided at the clinical setting, and may go to bed at a different time to their usual bedtime. Sleeping in an unfamiliar setting can also have an effect on the subject's sleep.

The unfamiliarity of the sleep setting (the "first night effect"), the unfamiliarity with the use of PAP therapy (e.g. sleeping while wearing a mask can result in claustrophobia and/or skin irritation if the subject is not used to it, and/or the subject may initially find it difficult to sleeping while receiving pressurized air), the discomfort of being connected to the wires needed for obtaining titration results (sensing vital signs and the physiological response to the pressure therapy), and the changes in the subject's behavior/lifestyle habits that occur during a PAP titration can mean that the subject's sleep during the titration study is unrepresentative of the subject's normal sleep when at home once the subject has become accustomed to PAP therapy. This can result in the subject being prescribed an inappropriate pressure setting for the PAP therapy, an inappropriate PAP therapy modality (e.g. a subject may be prescribed CPAP therapy when BiPAP or APAP therapy would be more appropriate for the subject) and/or an inappropriate patient interface.

PAP titration studies may also be carried out at a subject's home, but home titrations also involve changes to the subject's behavior/bedtime routine, so may again be unrepresentative of a normal night's sleep for the subject. Further, many home sleep tests do not provide an accurate apnea hypopnea index (AHI), as they are based on the total recording time rather than the total sleep time. Therefore, home titrations also often result in the subject being prescribed an inappropriate pressure setting and device selection.

Subjects undergoing PAP therapy with an inappropriate pressure setting, PAP therapy modality and/or patient interface for their normal sleep generally experience worsened sleep quality and, in many cases, choose not to continue with the therapy. Low adherence to PAP therapy is a particular problem in treating SDB conditions.

### SUMMARY OF THE INVENTION

The invention is defined by the claims.

According to examples in accordance with an aspect of the invention, there is provided a processing system for generating a predictive indicator representing a difference between a PAP titration sleep session and at least one conventional sleep session of a subject, the processing system being configured to: obtain titration data for the subject, wherein the titration data was acquired during the PAP titration sleep session and comprises at least one value for each of one or more sleep parameters and sleep influencing factor information; obtain conventional sleep data for the subject, wherein the conventional sleep data was acquired during the at least one conventional sleep session in which positive airway pressure, PAP, therapy was delivered by a PAP device, and wherein the conventional sleep data comprises at least one value for each of the one or more sleep parameters and sleep influencing factor information relating to one or more sleep influencing factors; process the titration data and the conventional sleep data to determine, if present, one or more differences between the PAP titration sleep session and the conventional sleep session; and generate a predictive indicator that indicates whether or not, if present, the one or more differences between the PAP titration sleep session and the at least one conventional sleep session satisfy a first predetermined condition.

By comparing data relating to the PAP titration sleep session and data relating to a conventional sleep session, discrepancies that may result in ineffective PAP therapy may be identified. Identifying one or more causes of ineffective therapy allows actions to improve an efficacy of the PAP therapy to be determined and taken (e.g. adjustments to the PAP therapy or to the behavior/environment of the subject) before the subject becomes discouraged and stops using the PAP device.

A conventional sleep session is a sleep session that occurs naturally in the subject's day-to-day life (e.g. sleeping at night at home).

The one or more sleep parameters may, for example, include a time and/or duration of a sleep stage, a number of arousals, a frequency of arousals, a number of SDB events, an apnea-hypopnea index, a duration of SDB events, and/or a type of SDB event.

The sleep influencing factor information may, for example, include a head position during sleep, a body position during sleep, a nasal obstruction, food intake, medication intake, alcohol intake, a mood assessment, a stress assessment, a time of going to bed, a time of waking, a type of mask used for PAP therapy, an air quality, an altitude, climate information, and/or other environmental information (e.g. room temperature, humidity, noise, light, a bed/mattress used).

In some examples, the step of processing the titration data and the conventional sleep data to determine, if present, the one or more differences between the PAP titration sleep session and the at least one conventional sleep session comprises: generating, for at least one value of at least one of the one or more sleep parameters in the titration data, a comparison result between said value and a corresponding value in the conventional sleep data, to thereby produce one or more comparison results; and in response to the one or more comparison results satisfying the first predetermined condition, processing the sleep influencing factor information in the titration data and the sleep influencing factor information in the conventional sleep data to determine, if present, one or more differences between sleep influencing factor information in the PAP titration sleep session and the at least one conventional sleep session.

In other words, one or more sleep parameters for the subject during the PAP titration are compared with the same one or more sleep parameters during a conventional sleep session. If a difference between the sleep parameters is observed, the sleep influencing factor information is compared in order to identify sleep influencing factors that could explain the difference in sleep parameters.

In some examples, in response to the first predetermined condition not being satisfied, the step of generating one or more comparison results is repeated at predetermined intervals (e.g. at predetermined intervals within a single sleep session, or at a subsequent sleep session).

In some examples, the sleep influencing factor information in the conventional sleep data comprises a subjective user assessment of the subject's sleep experience and information relating to one or more other sleep influencing factors; the sleep influencing factor information in the titration data comprises information relating to the one or more other sleep influencing factors; and the step of processing the sleep influencing factor information to determine, if present, the one or more differences comprises: processing the subjective user assessment of the subject's sleep experience to determine whether or not a second predetermined condition is satisfied; and in response to the second predetermined condition being satisfied, processing the information relating to the one or more other sleep influencing factors in the titration data and the information relating to the one or more other sleep influencing factors in the conventional sleep data to determine the one or more differences between the PAP titration sleep session and the at least one conventional sleep session.

The additional use of a subjective user assessment of the subject's sleep experience improves a robustness of the discrepancy determination. This recognizes that a single conventional sleep session of the subject is not necessarily representative of the subject's typical sleep.

For instance, drinking alcohol may affect the value of one or more sleep parameters, compared with the PAP titration sleep session for which the subject is required not to drink. However, if the subject does not drink frequently, then the PAP therapy pressure determined during the titration session may be appropriate for the majority of sleep sessions for the subject. A user assessment indicating that the subject is satisfied with their sleep and/or that the subject is comfortable with and adhering to the PAP therapy would indicate that this discrepancy may not be representative of the subject's typical sleep, and that the current PAP therapy may be appropriate for the subject.

In some examples, in response to the second predetermined condition not being satisfied, the steps of generating one or more comparison results and processing the user assessment to determine whether a second predetermined condition is satisfied are repeated at predetermined intervals (e.g. at predetermined intervals within a single sleep session, or at a subsequent sleep session).

In some examples, the processing system is further configured to control a first output user interface to provide a first user-perceptible output of the one or more differences.

This allows a clinician to determine an action to be taken to remedy the discrepancy (e.g. by making an adjustment to the therapy provided to the subject and/or advising the subject to change one or more aspects of their behavior and/or environment).

In some examples, the processing system is further configured to process the one or more differences between the PAP titration sleep session and the at least one conventional sleep session to determine a recommended action.

The recommended action may be an adjustment to the therapy provided to the subject (e.g. an adjustment to the pressure provided by the PAP device, a change to a different PAP device, and/or the use of an alternative therapy, such as positional therapy).

In some examples, the processing system is further configured to control a second output user interface to provide a second user-perceptible output of the recommended action.

The recommended action may be provided to the subject (e.g. if the recommended action is an adjustment to the subject's behavior and/or environment) or to a clinician (e.g. if the recommended action is an adjustment to the therapy provided to the subject).

In some examples, the recommended action comprises an adjusted pressure to be provided by the PAP device; and the processing system is further configured to generate a control signal configured to control the PAP device to provide air to the subject at the adjusted pressure.

In other words, the processing system may automatically adjust the pressure provided during PAP therapy to correct for the discrepancy between the PAP titration sleep session and the conventional sleep session.

In some examples, the step of determining a recommended action comprises: processing the one or more differences between the PAP titration sleep session and the at least one conventional sleep session to determine an initial recommended action; processing the titration data and the conventional sleep data to identify, for each of one or more aspects of the subject's behavior and/or environment, a relative effect of the aspect on a sleep quality for the subject; and processing the relative effect of each aspect and the initial recommended action to determine the recommended action.

In other words, the recommended action takes into account the effect the action would have on the quality of the subject's sleep. For instance, if the initial recommended action is to increase the air pressure provided by the PAP device, but an increase in pressure is determined to have a negative effect on the subject's sleep quality, the recommended action may be a smaller increase in the air pressure or an alternative to increasing the air pressure (e.g. the provision of supplementary oxygen).

In some examples, the processing system is further configured to: process the at least one value for at least one of the one or more sleep parameters in the titration data and/or the conventional sleep data to detect an arousal of the subject; process the titration data and/or the conventional sleep data to determine whether or not the detected arousal is caused by a sleep disordered breathing, SDB, event; and in response to a determination that the detected arousal is caused by an SDB event, determine an adjustment to be made to the PAP therapy provided to the subject.

If the subject is experiencing arousals caused by SDB events, this indicates that the PAP therapy being provided to the subject is not reducing SDB events sufficiently, and therefore that an adjustment should be made to the PAP therapy (e.g. increasing the pressure provided by the PAP device).

In some examples, in response to a determination that the detected arousal is not caused by an SDB event, the processing system is further configured to: process the titration data and/or the conventional sleep data to determine a cause of the detected arousal; and control a third output user interface to provide a third user-perceptible output of the cause of the detected arousal.

Arousals during the sleep session can result in the subject perceiving their quality of sleep as low. The subject may attribute this to the PAP therapy and decide to stop using the PAP device as a result. Informing the subject about the cause of arousals both allows the subject to make changes to their environment to reduce arousals, and corrects the subject's perception that the PAP device is negatively affecting their quality of sleep.

In some examples, the at least one value for each of the one or more sleep parameters in the conventional sleep data is acquired by one or more sleep parameter sensors comprising at least one of: an airflow sensor, a pressure sensor, a cardiorespiratory sensor, a brain activity sensor, a muscle sensor, and/or a pulse oximetry sensor; and the sleep influencing factor information in the conventional sleep data is acquired by one or more sleep influencing factor sensors comprising at least one of: a user input device, an accelerometer, a particle sensor and/or an exhaled breath sensor.

In some examples, the conventional sleep data comprises data acquired during a plurality of conventional sleep sessions.

The use of data from multiple sleep sessions recognizes that a single sleep session may not be representative of typical sleep for the subject, and allows lifestyle variations (e.g. different behaviors during the week and at weekends) to be identified and taken into account.

There is also provided a positive airway pressure, PAP, device comprising: one or more sleep parameter sensors configured to acquire at least one value for each of one or more sleep parameters; one or more sleep influencing factor sensors configured to acquire sleep influencing factor information; and the processing system described above.

Since the subject is already required to use the PAP device for PAP therapy, the use of sensors in the PAP device allows conventional sleep data to be obtained without the addition of further equipment, which might increase the subject's discomfort.

For instance, the one or more sensors may include the airflow sensor and/or pressure sensor that are typically found in PAP devices. Additional sensors may, for instance, be embedded in the mask of the PAP device.

Additionally and/or alternatively, one or more sensors may be comprised in a separate wearable device, such as a chest belt, an abdominal belt, a snore relief band, and/or a positional therapy device, and/or in a nearable device.

According to another aspect of the invention, there is provided a computer-implemented method for generating a predictive indicator representing a difference between a PAP titration sleep session and at least one conventional sleep session of a subject, the computer-implemented method comprising: obtaining titration data for the subject, wherein the titration data was acquired during the PAP titration sleep session and comprises at least one value for each of one or more sleep parameters and sleep influencing factor information relating to one or more sleep influencing factors; obtaining conventional sleep data for the subject, wherein the conventional sleep data was acquired during the at least one conventional sleep session in which positive airway pressure, PAP, therapy was delivered by a PAP device, and wherein the conventional sleep data comprises at least one value for each of the one or more sleep parameters and sleep influencing factor information; processing the titration data and the conventional sleep data to determine, if present, one or more differences between the PAP titration sleep session and the at least one conventional sleep session; and generating a predictive indicator that indicates whether or not, if present, the one or more differences between the PAP titration sleep session and the at least one conventional sleep session satisfy a first predetermined condition.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for generating a predictive indicator representing a difference between a PAP titration sleep session and at least one conventional sleep session of a subject, according to an embodiment of the invention;
Fig. 2 illustrates a computer-implemented method for generating a predictive indicator representing a difference between a PAP titration session and at least one conventional sleep session of a subject, according to an embodiment of the invention; and
Fig. 3 illustrates an example method for performing a step of processing titration data and conventional sleep data in the method of Fig. 2.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for generating a predictive indicator representing a difference between a PAP titration sleep session and at least one conventional sleep session. Data relating to the subject's sleep and to sleep influencing factors is obtained for both the PAP titration sleep session and the conventional sleep session, and is processed to identify any differences between the PAP titration sleep session and the conventional sleep session. A predictive indicator indicative of whether the identified difference(s), if present, satisfy a first predetermined condition is generated.

Embodiments are at least partly based on the realization that differences in sleep influencing factor information between the PAP titration sleep session and the subject's conventional sleep may affect the subject's sleep, which in turn may affect the subject's perception of the PAP therapy and their adherence to the PAP therapy. By identifying differences between the PAP titration sleep session and the conventional sleep session, action to improve the subject's sleep experience and therapy adherence may be taken.

Illustrative embodiments may, for example, be employed in PAP therapy systems.

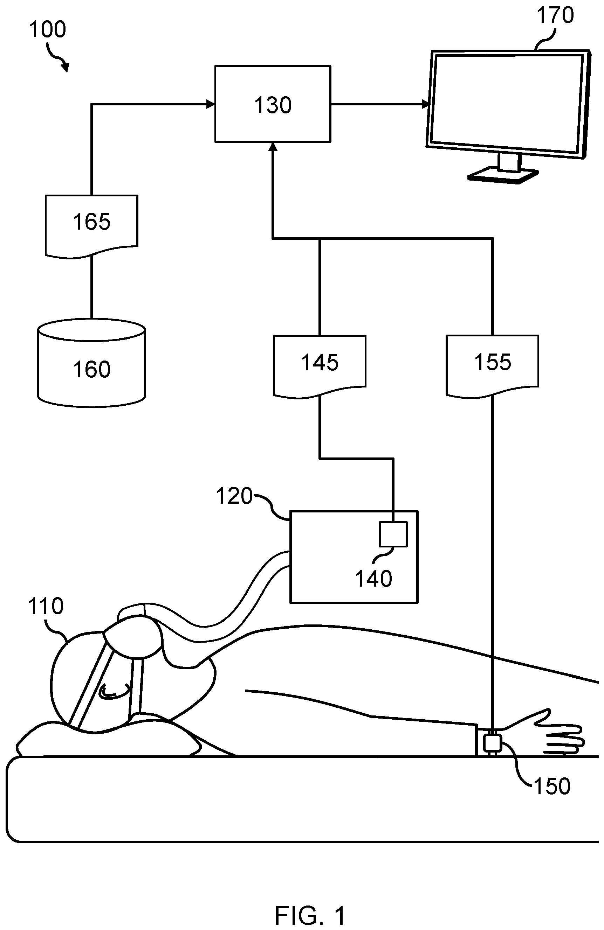

Fig. 1 illustrates a system 100 for generating a predictive indicator representing a difference between a PAP titration sleep session and at least one conventional sleep session of a subject 110, according to an embodiment of the invention. The system 100 comprises a positive airway pressure (PAP) device 120, a processing system 130, one or more sleep parameter sensors 140, one or more sleep influencing factor sensors 150, and a memory unit 160. The processing system is, itself, an embodiment of the invention.

The PAP device 120 may be any type of PAP device (e.g. a CPAP device, BiPAP device or APAP device), and comprises a PAP machine that generates pressurized air, a mask that fits over the subject's nose and/or mouth, and a tube connecting the mask to the PAP machine.

The processing system 130 is shown as separate from the PAP device 120 in Fig. 1. However, as the skilled person will readily appreciate, the processing system may alternatively be provided in the PAP device 120.

A conventional sleep session is a sleep session in which the subject is sleeping "normally" (i.e. a sleep session for which the purpose is simply to sleep, as opposed to a PAP titration sleep session, which is carried out in order to determine a PAP therapy prescription). The at least one conventional sleep session may, for example, take place at night in the subject's home. Preferably, the at least one conventional sleep session occurs once the subject has, at least partly, adapted to the PAP therapy (e.g. after a few nights of undergoing PAP therapy), in order to reduce the effect of unfamiliarity with the therapy on the subject's sleep.

A PAP titration sleep session is a sleep session during which a PAP titration is carried out. The PAP titration may take place in a dedicated titration setting (e.g. a clinical setting, such as a sleep laboratory or hospital, or a specially-adapted hotel room) or in the subject's home.

The PAP titration sleep session differs principally from conventional sleep sessions in that the pressure provided by the PAP device is gradually increased, while the subject is monitored, in order to determine an appropriate pressure for PAP therapy to be prescribed to the subject. The PAP titration sleep session also differs from the conventional sleep session in that more types of sensors for monitoring the subject's sleep are used during the PAP titration sleep session (i.e. the sleep parameter sensors used to acquire conventional sleep data during the at least one conventional sleep session are a subset of the sensors used in a PAP titration sleep session). In some examples, the PAP titration sleep session may further differ from the conventional sleep session in that the PAP titration sleep session takes place in a clinical setting. PAP titration sleep sessions that are carried out in a home setting may differ from conventional sleep sessions in that the range of air pressure permitted during a PAP titration sleep session is typically wider, in order to allow the titration system to determine an appropriate pressure for the subject.

The one or more sleep parameter sensors 140 and the one or more sleep influencing factor sensors 150 are together configured to acquire conventional sleep data for the subject 110 during at least one conventional sleep session in which PAP therapy is delivered to the subject by the PAP device 120. Preferably, the conventional sleep data is acquired for a plurality of sleep sessions, in order to provide a more robust comparison between the PAP titration sleep session and the subject's normal sleep.

The one or more sleep parameter sensors 140 are configured to acquire at least one value for each of one or more sleep parameters 145 for the subject 110. A sleep parameter, in the context of the present disclosure, is a parameter representative of a sleep architecture, sleep quality or breathing during sleep. Suitable sleep parameters include, for example, a time and/or duration of a sleep stage, a number of arousals, a frequency of arousals, a number of sleep disordered breathing (SDB) events, an apnea-hypopnea index (AHI), a duration of SDB events, a type of SDB event, and/or an indication as to whether the subject has insomnia. Further examples of suitable sleep parameters will be apparent to the skilled person.

The one or more sleep parameter sensors 140 used to acquire the value(s) for the sleep parameter(s) may depend on the particular sleep parameter(s) that is/are acquired. For instance, sleep parameters relating to sleep architecture and/or sleep quality may be obtained by one or more airflow sensors (e.g. the airflow sensor(s) comprised in a PAP device), one or more pressure sensors (e.g. the pressure sensor(s) comprised in a PAP device), one or more cardiorespiratory sensors (e.g. an electrocardiography (ECG) sensor, a photoplethysmography (PPG) sensor, a ballistocardiography (BCG) sensor, a seismocardiography (SCG) sensor, etc.), one or more brain activity sensors (e.g. an electroencephalography (EEG) sensor), one or more muscle activity sensors (e.g. an electromyography (EMG) sensor), one or more body and/or head posture sensors, one or more accelerometers, and/or electrooculography (EOG) sensors. Sleep parameters relating to the subject's breathing during sleep may, for example, be obtained by an SpO2 sensor (i.e. a pulse oximeter, such as a PPG sensor) and/or an airflow sensor. Further examples of suitable sensors for acquiring values for each sleep parameter will be apparent to the skilled person.

The use of the above-mentioned sensors to acquire values of sleep parameters relating to sleep architecture, sleep quality and/or breathing during sleep is known, and suitable techniques for determining values for the one or more sleep parameters will be apparent to the skilled person. See, for example: Li et al. (2021), "DeepSleep convolutional neural network allows accurate and fast detection of sleep arousal", Communications Biology, 4:18; Nam et al. (2016), "Sleep monitoring based on a tri-axial accelerometer and a pressure sensor", Sensors, 16(5):750; Bakker et al. (2021), "Estimating sleep stages using cardiorespiratory signals: validation of a novel algorithm across a wide range of sleep-disordered breathing severity", J Clin Sleep Med, 17(7):1343-1354; Lee et al. (2022), "Applications of wireless sensor systems to sleep stage estimation for home sleep monitoring", IET Wireless Sensor Systems, 12(5-6): 123-133; Imtiaz (2021), "A systematic review of sensing technologies for wearable sleep staging", Sensors, 21(5): 1562; Nakano et al. (2007), "Automatic detection of sleep-disordered breathing from a single-channel airflow record", European Respiratory Journal, 29:728-736; and John et al. (2021), "SomnNET: An SpO2 based deep learning network for sleep apnea detection in smartwatches", 2021 43rd Annual International Conference of the IEEE Engineering in Medicine & Biology Society, 1961-1964.

The one or more sleep influencing factor sensors 150 are configured to acquire sleep influencing factor information 155 for the subject 110. Sleep influencing factor information is information relating to one or more sleep influencing factors that may affect the subject's sleep, and therefore may affect an efficacy of the PAP therapy. For example, sleep influencing factors may include aspects of the subject's lifestyle, aspects of the subject's sleep behavior and/or aspects of the subject's sleeping environment.

For instance, the sleep influencing factor information 155 may comprise one or more of: a head position during sleep, a body position during sleep, a movement during sleep, a nasal obstruction, food intake, alcohol intake, a drug intake, a mood assessment, a stress assessment, a time of going to sleep, a time of waking, information relating to circadian rhythm (e.g. an estimated current circadian phase, a time of minimum core body temperature and/or a time of dim-light melatonin onset), a type of mask used for PAP therapy, an adherence to the PAP therapy, an air quality, an altitude, climate information, a room temperature, a humidity, a noise level, a light level, and/or a bed and/or mattress used by the subject for the sleep session. The sleep influencing factor information may additionally comprise at least one subjective user assessment of the subject's sleep experience, as the subject's perception of their sleep experience can affect the subject's sleep. The at least one subjective user assessment of the subject's sleep experience may comprise a user assessment of sleep quality and/or a user assessment of a difficulty with PAP therapy adherence (e.g. whether the user is struggling to use the PAP device for the prescribed number of hours a night).

The one or more sleep influencing factor sensors 150 may depend on the particular sleep influencing factor(s) included in the sleep influencing factor information. For instance, an accelerometer may provide a head position during sleep, a body position during sleep and/or a movement during sleep.

A user input device may be used to obtain information relating to food intake, alcohol intake, a drug intake and/or one or more subjective user assessments (e.g. a mood assessment, a stress assessment, and/or a subject user assessment of the subject's sleep experience).

An exhaled breath sensor may be used to obtain information relating to food intake, alcohol intake and/or drug intake.

A particle sensor (e.g. a gas sensor) may be used to obtain information relating to air quality.

A nasal obstruction may be identified by identifying reduced outflow of air from the nostrils using a pressure and/or airflow sensor provided in the mask of the PAP device, or by acoustical reflectometry (rhinometry). In some examples, additional information (e.g. obtained via user input, a pollen count from a regional weather service or an air quality sensor) may be used to determine whether an identified nasal obstruction is due to a full or partial obstruction of the airways or due to some other cause (e.g. hay fever or a virus such as a cold or flu), in order to eliminate obstructions having other causes. For instance, an obstruction in only one nostril (with good airflow through the other nostril) may indicate that the nasal obstruction is not due to a general obstruction of the airways. Similarly, user input and/or air quality information indicating a high pollen count may be used to identify nasal obstructions due to hay fever.

A time of going to sleep and/or a time of waking may be determined based on data from the one or more sleep parameter sensors 140.

Information relating to circadian rhythm may be determined based on core body temperature measurements, actigraphy data (e.g. to determine active/rest cycles), melatonin concentration, data from an EEG sensor, and/or data from the one or more sleep parameter sensors (e.g. by determining a sleep stage).

Information relating to mask usage (e.g. a type of mask) may be determined based on radio-frequency identification (RFID), near-field communication (NFC) technology, flow parameters measured by the airflow sensor of the PAP device 120 and/or user input. Suitable methods for determining information relating to mask usage will be apparent to the skilled person: see, for example, European Patent No. EP 2,379,151 B1, US Patent Nos. US 10,279,134 B2, US 7,913,689 B2 and US 9,199,048 B2, and US Patent Application US 2010/0147301 A1.

Information relating to an adherence to the PAP therapy may be obtained from usage data collected by the PAP device.

Altitude and/or climate information may be obtained via user input, or may be derived from location information obtained by a location sensor (e.g. a GPS sensor).

A microphone, thermometer, radar sensor and/or camera may be used to obtain information relating to the sleeping environment.

In Fig. 1, the sleep parameter sensor(s) 140 are provided within the PAP device 120, while the sleep influencing factor sensor(s) 150 are provided in a smartwatch worn by the subject. Each of the sleep parameter sensor(s) 140 and sleep influencing factor sensor(s) 150 may, as appropriate, be provided in any of: the PAP device 120 (e.g. in a mask of the PAP device or, particularly in the case of airflow and/or pressure sensors, in the PAP machine), a wearable device separate from the PAP device (e.g. a smartwatch, a chest belt, an abdominal belt, a snore relief band, a positional therapy device, etc.), or a nearable device (e.g. a smartphone).

The memory unit 160 stores titration data 165 for the subject 110. The titration data is data that was acquired during a PAP titration sleep session for the subject, and comprises at least one value for each of one or more sleep parameters and sleep influencing factor information for the titration session. The one or more sleep parameters in the titration data comprise at least some of the sleep parameter(s) included in the conventional sleep data, and the sleep influencing factor comprises information relating to at least some of the sleep influencing factor(s) included in the conventional sleep data, in order to allow a comparison to be made between the titration data and the conventional sleep data.

Values for sleep parameters are routinely acquired during a PAP titration sleep session, in order to make a determination as to an appropriate pressure at which to provide PAP therapy.

For some sleep influencing factors, such as a position and/or movement during sleep, information for the PAP titration sleep session may be recorded during the titration session. Information for other sleep influencing factors may be obtained from clinical requirements of the PAP titration sleep session: this is particularly the case where the PAP titration session occurs in a clinical setting, where factors such as food intake, alcohol intake, a time of going to sleep, a time of waking, and a type of mask used may be regulated by the clinical setting. Where the PAP titration sleep session takes place at home, the one or more sleep parameter sensors 140 and the one or more sleep influencing factor sensors may be used to obtain the titration data 165.

In some examples, the conventional sleep data 145, 155 may also be stored by the memory unit 160 before being processed. In other examples, the conventional sleep data may be processed as it is acquired.

The processing system 130 is configured to obtain the titration data 165 and the conventional sleep data 145, 155, and to process the titration data and the conventional sleep data to determine, if present, one or more differences between the PAP titration sleep session and the at least one conventional sleep session. Methods for determining differences between data are well known, and suitable techniques will be readily apparent to the skilled person.

Having determined one or more differences between the PAP titration sleep session and the at least one conventional sleep session, the processing system 130 is configured to generate a predictive indicator that indicates whether or not, if present, the one or more differences between the PAP titration sleep session and the at least one conventional sleep session satisfy a first predetermined condition.

The first predetermined condition may, for example, relate to differences in the sleep parameter(s). For instance, the first predetermined condition may require that a difference between a value for a sleep parameter exceeds a predetermined sleep parameter threshold (the value of which would depend on the sleep parameter in question). In other words, the first predetermined condition is satisfied only if the subject experiences a sufficient change in their sleep architecture, sleep quality and/or breathing during sleep during conventional sleep compared with the PAP titration sleep session. This enables situations in which differences in sleep influencing factors have a significant effect on the subject's sleep to be distinguished from situations in which these differences have no significant effect on the subject's sleep.

In some examples, the processing system 130 may process all of the titration data and all of the conventional sleep data to determine any differences in a single step. In other examples, the comparison between the titration data and the conventional sleep data may comprise more than one sub-step. For instance, the processing system may be configured to first compare the sleep parameter(s), generating a comparison result between at least one value of at least one sleep parameter in the titration data and a corresponding value in the conventional sleep data. The processing system may then be configured to process the sleep influencing factor information in the titration data and in the conventional sleep data to identify differences in the sleep influencing factor information in response to the comparison result(s) satisfying the first predetermined condition.

In other words, the processing system 130 may, in some examples, only determine differences between the sleep influencing factor information in the titration data and the sleep influencing factor information in the conventional use data if there is a difference in the sleep parameters that satisfies the first predetermined condition.

In response to the comparison result(s) failing to satisfy the first predetermined condition, the processing system 130 may be configured to repeat the step of generating the comparison result(s) between the sleep parameter(s) at predetermined intervals until the first predetermined condition is satisfied, or until a predetermined maximum number of repetitions have occurred, in order to account for variations in the subject's sleep. The predetermined interval may be an interval within a sleep session (e.g. every hour during the at least one conventional sleep session), or may be an interval measured in sleep sessions (e.g. the comparison result(s) may be generated every conventional sleep session or every other conventional sleep session).

In some examples, the sub-step of processing the sleep influencing factor information in the titration data and in the conventional sleep data to identify differences may comprise more than one sub-step. For instance, where the sleep influencing factor information in the conventional sleep data comprises a subjective user assessment of the subject's sleep experience and information relating to one or more other sleep influencing factors, the processing system may be configured to first process the subjective user assessment of the subject's sleep experience to determine whether or not a second predetermined condition is satisfied, and process the information relating to the one or more other sleep influencing factors in the titration data and the conventional sleep data only in response to the second predetermined condition being satisfied.

The second predetermined condition may be satisfied if the subjective user assessment of the subject's sleep experience indicates that the subject has a negative experience of sleep. In other words, once the first predetermined condition relating to the difference(s) in sleep parameters is satisfied, the subject's own assessment of their sleep experience is considered before other sleep influencing factors are compared. In response to an indication that the subject has a negative experience of sleep, the other sleep influencing factors are compared, while in response to an indication that the subject has a positive experience of sleep, the other sleep influencing factors are not compared. This reduces a risk of making unnecessary adjustments to the subject's PAP therapy in the case that the subject is satisfied with their sleep.

The determination of whether the subjective user assessment of the subject's sleep experience indicates a positive or a negative experience of sleep will depend on the nature of the subjective user assessment, and suitable methods for determining this will be apparent to the skilled person. For example, if the subjective user assessment involves the subject providing one or more numerical ratings, the second predetermined condition may comprise a numerical threshold. If the subjective user assessment involves free text input, a natural language processing algorithm may be used to determine whether the second predetermined condition is satisfied.

In some examples, the processing system 130 is configured to control a first output user interface 170 to provide a user-perceptible output of the generated predictive indicator. In response to one or more differences being present and satisfying the first predetermined condition, the processing system may control the first output user interface to provide a user-perceptible output of the one or more differences. In response to a determination that no differences are present, or that no differences satisfy the first predetermined condition, the processing system may control the first output user interface to provide a user-perceptible output indicating that there are no (significant) differences between the PAP titration sleep session and the at least one conventional sleep session.

Suitable user-perceptible outputs will be apparent to the skilled person, and may depend on the control instruction provided. For instance, the difference(s) may be provided to the user in the form of a textual display, an image, and/or a sound. In Fig. 1, the first output user interface 170 is a computer monitor; however, the skilled person will readily appreciate that any output user interface capable of providing a suitable user-perceptible output may be used (e.g. a mobile device or a speaker). The user-perceptible output may be provided to a clinician or caregiver of the subject, or to the subject; the user provided with the user-perceptible output may depend on the content of the user-perceptible output.

In some examples, the processing system 130 may additionally or alternatively be configured to process the one or more differences (if present) between the PAP titration sleep session and the at least one conventional sleep session to determine a recommended action. In response to a determination that no differences between the PAP titration sleep session and the at least one conventional sleep session have been identified (or, where relevant, in response to a failure to satisfy the first predetermined condition and/or the second predetermined condition), the processing system may not determine a recommended action, or may recommend that no adjustments are made to the PAP therapy.

In some examples, the processing system 130 may determine a recommended action based on one or more predetermined rules. The predetermined rules may, for example, be determined based on standard clinical protocols, or be defined by a clinician of the subject 110. In other examples, the processing system 130 may determine a recommended action using a machine-learning algorithm trained process one or more difference(s) between a PAP titration session and at least one conventional sleep session to output a recommended action. The machine-learning algorithm may be trained using population data or subject-specific data.

In some examples, the processing system 130 may determine more than one recommended action (e.g. the recommended action may be a list of alternatives, or a number of actions to be performed together).

The determined recommended action may relate to an adjustment to the PAP therapy provided to the user and/or to an adjustment to the subject's lifestyle, behavior and/or environment. Recommended actions relating to an adjustment to the PAP therapy provided to the user may, for example, include one or more of: an adjustment to the pressure provided by the PAP device, a change in PAP therapy modality (e.g. a change from CPAP to BiPAP or APAP), and/or a change in the subject interface (e.g. a change in the type of mask used, or an adjustment to the mask fit).

As the skilled person will appreciate, the determined recommended action will depend on the difference(s) identified between the PAP titration sleep session and the at least one conventional sleep session. Some non-limiting examples of recommended actions for specific scenarios are provided below as guidance; the skilled person will be readily capable of adapting these non-limiting examples to other scenarios.

For instance, if a comparison between the titration data and the conventional sleep data indicates that the subject spends substantially less time sleeping in a supine position during the at least one conventional sleep session than in the PAP titration sleep session, and that the subject is struggling with the PAP therapy (e.g. based on PAP device use data indicating declining adherence or irregular patterns of use, or based on a subjective user assessment of a difficulty of PAP therapy adherence), the processing system may determine that the pressure provided by the PAP device is too high for the subject's conventional sleeping position.

In this case, the determined recommended action may comprise one or more of: re-titrating the subject while the subject sleeps in a position more typical of their conventional sleep; changing the subject's PAP therapy modality to an auto-PAP modality, in which the pressure is automatically adjusted according to sleeping position; and/or providing combination therapy, in which the PAP therapy is provided at a lower pressure in combination with positional therapy to guide the subject to a non-supine sleeping position in which the lower pressure is sufficient.

In another example, the comparison between the PAP titration sleep session and the at least one conventional sleep session may indicate that the subject experienced more and/or longer periods of REM sleep during the at least one conventional sleep session, e.g. because the subject's discomfort and unfamiliarity with the PAP equipment during the PAP titration sleep session results in frequent arousals that prevent a continued, stable REM period, or because the irregular breathing of REM sleep was mistakenly identified as sleep-disordered breathing during titration. This is particularly likely to occur where the PAP titration session took place at the subject's home using an auto-titration system, but may also occur in clinical settings, particularly if the PAP titration sleep session is carried out for only part of a night.

If this is the case and the comparison further indicates that the subject's AHI during REM exceeds a predetermined threshold, and that the PAP therapy is not effective at alleviating their daytime symptoms of their SDB condition (or that the subject has such a perception), the processing system may determine that the pressure provided by the PAP device is insufficient during REM sleep. The determined recommended action may then comprise one or more of: re-titrating the subject, preferably in a clinical setting, while ensuring that the subject's REM sleep is more representative of the at least one conventional sleep session; increasing the overall pressure provided by the PAP device to account for the increased pressure required during REM sleep (e.g. if this does not result in an excessively high pressure during non-REM sleep); and/or increasing the pressure provided by the PAP device only during REM sleep (which may include a recommendation that the subject's PAP therapy modality is changed to an auto-PAP modality, e.g. if the subject's current PAP therapy modality is CPAP).

In yet another example, the subject may have an increasing intolerance to expiratory pressure in the later stages of a sleep session (e.g. in the second half of a night), increasing a likelihood of arousal. This may not be identified in a PAP titration sleep session that is carried out only in the first half of a night. Therefore, if the comparison between the PAP titration sleep session and the at least one conventional sleep session indicates that the subject is experiencing a greater number/frequency of arousals during the at least one conventional sleep session, particularly if the comparison indicates that the number of arousals increases later in the sleep session, the recommended action may comprise changing the PAP therapy modality to BiPAP, in which different pressure levels are used for inspiration and expiration).

In yet another example, in which the at least one conventional sleep sessions comprise a plurality of conventional sleep sessions, the comparison between the PAP titration sleep session and the conventional sleep sessions may indicate that the subject has significant lifestyle variations at the weekend compared with during the week (e.g. variations in a time of going to sleep, alcohol intake etc.), and compared with the PAP titration sleep session. If these differences coincide with differences in the subject's AHI and sleep architecture, the determined recommended action may comprise re-titrating the subject at home during a weekend night to determine "weekend settings" for the PAP therapy and/or changing the subject's PAP therapy modality to an auto-PAP modality. "Weekend settings" may, for instance, be used automatically at weekends and/or in response to a determination that the subject is exhibiting "weekend behavior" (e.g. drinking alcohol and/or going to sleep later than usual).

In yet another example, the comparison between the PAP titration sleep session and the at least one conventional sleep session may indicate that the subject has changed to a different mask. This can result in a different level of pressure being provided to the subject, due in part to a different amount of mask leakage. If a change in mask is detected, and the comparison further indicates a difference in AHI compared with the PAP titration session and/or an change in therapy adherence, the determined recommended action may comprise re-titrating the subject while the subject wears the new mask and/or changing the subject's mask, either to the original mask or to another mask with an improved fit (in which case, a re-titration with the new mask may also be recommended).

In yet another example, the comparison between the PAP titration sleep session and the at least one conventional sleep session may indicate a difference in one or more sleep parameters and an increase in a stress level of the subject (and/or a decrease in a wellbeing of the subject). Stressful situations, such as the loss of a partner or family member, may have a direct effect on the subject's sleep and/or may cause changes to the subject's behavior that result in changes to the subject's sleep (e.g. an increase in alcohol intake). In this case, the recommended action may comprise one or more of: coaching the subject to help the subject cope with their stressful situation, assessing the subject for insomnia, and/or providing cognitive behavioral therapy to address the subject's insomnia (if identified).

In some examples, an expected effect of the recommended action may be taken into account when determining the recommended action. For instance, the processing system 130 may be configured to determine a recommended action by processing the difference(s) between the PAP titration sleep session and the at least one conventional sleep session, as described above, to determine an initial recommended action. The processing system may also process the titration data and/or the conventional sleep data to identify, for each of one or more aspects of the subject's behavior and/or environment, a relative effect of the aspect on a sleep quality of the subject.

The one or more aspects of the subject's behavior and/or environment may, for example, comprise one or more of: a pressure provided to the subject by the PAP device, wearable sensors and/or wires worn by the subject, a subject interface worn by the subject (e.g. a mask of the PAP device), a temperature of the sleeping environment, a noise level of the sleeping environment, a diet of the subject, an alcohol intake of the subject, a physical activity level of the subject, a time of going to sleep, a time of waking up, a presence of a bedpartner and/or psychological aspect. Further suitable aspects may be apparent to the skilled person. Information relating to these aspects may be comprised in the sleep influencing factor information in the titration data and conventional sleep data.

The processing system 130 may identify a relative effect of one or more of these aspects by processing the titration data and/or the conventional sleep data to identify how a change in each aspect has previously affected the subject's sleep quality. For instance, the titration data and/or conventional sleep data may indicate that the subject's sleep quality decreases when the subject wears more wearable sensors/wires, when the pressure provided by the PAP device increases above a certain level, when the temperature of the sleeping environment is too high or too low, when the subject's alcohol intake is higher, when the subject's physical activity level is lower, and/or when the subject's bedpartner is present or missing.

The processing system 130 may then process the relative effect of each aspect and the initial recommended action to determine the recommended action. For example, the processing system may determine the recommended action to be the initial recommended action in response to a determination that the aspect(s) of the subject's behavior and/or environment to which the initial recommended action relates do not have a negative effect on the subject's sleep quality. In response to a determination that the aspect(s) do have a negative effect on the subject's sleep quality, the processing system may modify the initial recommended action to determine the recommended action.

For instance, the titration data and/or the conventional sleep data may indicate that an increase in pressure provided by the PAP device has previously resulted in an increase in the number of arousals experienced by the subject. If this is the case, and the initial recommended action relates to an increase in the pressure provided by the PAP device, the processing system may instead determine as the recommended action a smaller increase in pressure than would otherwise be recommended, or may recommend an alternative adjustment to the PAP therapy that does not involve increasing the pressure.

For instance, suitable alternatives to increasing the pressure may include: changing to an alternative PAP therapy modality (e.g. changing from CPAP to BiPAP); providing different pressure settings for inspiration and expiration, with pressure relief provided at the start of the expiration cycle; changing to a different subject interface (e.g. from a full face mask to a nasal mask/nasal pillow) and/or providing another type of SDB therapy (e.g. positional therapy or jaw repositioning/stabilization) in combination with the PAP therapy (combination therapy). Which of these alternatives may be recommended would depend on characteristics of the subject: for instance, positional therapy would be a suitable recommendation for subjects that typically sleep in a supine position, and a switch to a nasal mask would be a suitable recommendation for subjects who breathe through their nose (but would not be a suitable recommendation for mouth breathers).

The processing system 130 may be configured to control a second output user interface to provide a user-perceptible output of the recommended action. The second output user interface may be the same user interface as the first output user interface, or may be a different user interface. For example, the first output user interface may provide a user-perceptible output to a clinician or caregiver (e.g. a user-perceptible output of the one or more differences and/or of a recommended action relating to an adjustment to the therapy provided to the subject), and the second output user interface may provide a user-perceptible output to the subject (e.g. a user-perceptible output of a recommended action relating to an adjustment of the user's lifestyle, sleep behavior and/or sleeping environment).

In some examples, the processing system 130 may, as an alternative or in addition to providing a user-perceptible output of the recommended action, generate a control signal configured to put the recommended action into effect. This may be the case when the recommended action relates to an action that the processing system is able to control directly: for instance, where the recommended action comprises an adjusted pressure to be provided by the PAP device 120, the processing system may generate a control signal configured to control the PAP device to provide air to the subject at the adjusted pressure.

In some examples, for instance, in cases in which an appropriate adjusted pressure can be determined with a high level of confidence (e.g. used a trained, subject-specific machine-learning algorithm), a single adjustment may be made to the pressure provided by the PAP device 120. In other examples, the processing system 130 may control the PAP device to iteratively modify the pressure provided to the subject until a predetermined condition is met (e.g. until the identified difference(s) in the sleep parameter(s) falls below a predetermined threshold).

In some examples, there may be one or more predefined limits on automatic changes to the pressure. For instance, the processing system 130 may only directly control the PAP device 120 to provide air to the subject at an adjusted pressure in response to the adjusted pressure falling within a predefined range. In response to the adjusted pressure failing to fall within the predefined range, the processing system 130 may instead control an output user interface to provide a user-perceptible output of the adjusted pressure, thus allowing a clinician to make a decision on whether a large change to the pressure should be made.

In some examples, the processing system 130 is further configured to detect an arousal of the subject during the PAP titration sleep session and/or the at least one conventional sleep session, and to respond according to a cause of the detected arousal.

The processing system 130 may detect an arousal of the subject by processing at least one value for at least one of the one or more sleep parameters in the titration data and/or the conventional sleep data. Methods for detecting an arousal based on one or more sleep parameters are known, as described above.

Having detected an arousal, the processing system may process the titration data and/or the conventional sleep data to determine whether or not the detected arousal is caused by a sleep-disordered breathing (SDB) event. In particular, the processing system may process the titration data and/or the conventional sleep data to detect an SDB event: in response to a detected SDB event corresponding to the detected arousal, the processing system determines that the detected arousal is caused by the SDB event. In response to a failure to detect an SDB event or a determination that the detected arousal does not correspond to a detected SDB event, the processing system determines that the detected arousal is not caused by an SDB event. An SDB event may be considered to correspond to a detected arousal if the arousal occurs within a predetermined time interval of the SDB event. Typically, an arousal caused by an SDB event occurs at the end of or after an SDB event. The length of the predetermined time interval may depend on a type of SDB event detected: for instance, arousals caused by unresolved SDB events can occur up to 10-15 seconds after the event. Arousals caused by obstructive events tend to occur faster than this (typically within a couple of seconds), while arousals caused by central events can take longer to occur.

In some examples, the processing system 130 may, in response to a determination that the detected arousal is caused by an SDB event, determine an adjustment to be made to the PAP therapy provided to the subject (e.g. an adjustment to the pressure provided by the PAP device or a change in PAP therapy modality).

The processing system may control an output user interface to provide a user-perceptible output of the determined adjustment, or directly control the PAP device to make the adjustment. In some examples, the processing system may determine whether to provide a user-perceptible output or directly control the PAP device based on one or more predetermined rules (e.g. a user-perceptible output may be provided when the determined adjustment relates to changing a PAP therapy modality, while a determined adjustment comprising a slight adjustment to the pressure provided by the PAP device may be made by directly controlling the PAP device).

In some examples, the processing system 130 may, in response to a determination that the detected arousal is not caused by an SDB event, determine a cause of the detected arousal by processing the titration data and/or the conventional sleep data. The processing system may then control a third output user interface (which may be the same user interface as the first and/or second user interface(s)) to provide a user perceptible output of the cause of the detected arousal.

Possible causes of arousal other than SDB events include environmental causes (e.g. noise levels, light levels, etc.), behavioral causes (e.g. irregular sleep habits, alcohol intake, drug intake, etc.), physiological causes (e.g. baroreceptor activation due to a high PAP pressure, or chemoreceptor activation caused by oxygen desaturation and/or increased CO₂ in the bloodstream) and inappropriate PAP therapy settings (e.g. an inappropriate pressure, an inappropriate PAP therapy modality and/or an inappropriate subject interface).

Noise may be determined as a cause of a detected arousal if the sensors 140, 150 comprise one or more microphones, and a noise is detected by the one or more microphones shortly before the detected arousal occurs. Limb movements (e.g. detected by an EMG sensor, bed sensor, doppler radar, accelerometer, gyroscope, etc.) occurring close to a time of arousal may indicated that the arousal is movement-related. If arousals predominantly occur during the expiratory respiratory cycle, this may indicate that the arousals are caused by an excessively high expiratory pressure. If arousals predominantly occur during a particular sleep stage, particularly during sleep stages in which the arousability threshold is lower, or if arousals occur predominantly during the second half of the sleep session, this may indicate that the arousals are caused by inappropriate PAP therapy settings. If a frequency of arousals increases after an adjustment to the PAP therapy, this may indicate that the adjustment to the PAP therapy is causing an increase in arousals.

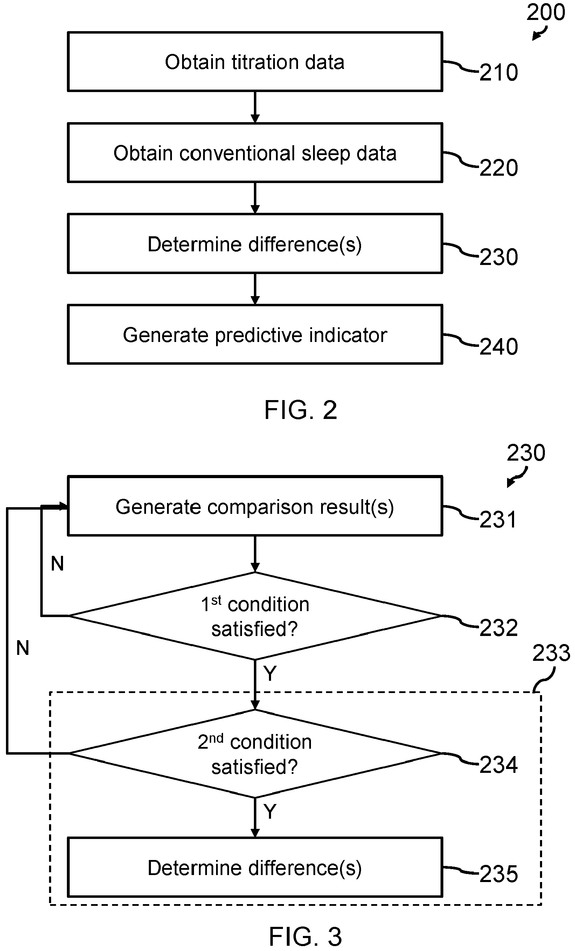

Fig. 2 illustrates a computer-implemented method 200 for generating a predictive indicator representing a difference between a PAP titration session and at least one conventional sleep session of a subject, according to an embodiment of the invention.

The method begins at step 210, at which titration data for the subject is obtained. The titration data is data acquired during a PAP titration sleep session, and comprises at least one value for each of one or more sleep parameters, and sleep influencing factor information.

At step 220, conventional sleep data for the subject is obtained. Conventional sleep data is data acquired during at least one conventional sleep session in which PAP therapy was delivered by a PAP device, and comprises at least one value for each of the one or more sleep parameters, and sleep influencing factor information.

At step 230, the titration data and conventional sleep data is processed to determine, if present, one or more differences between the PAP titration sleep session and the at least one conventional sleep session.

At step 240, a predictive indicator is generated. The predictive indicator indicates whether or not, if present, the one or more differences between the PAP titration sleep session and the at least one conventional sleep session satisfy a first predetermined condition.

Fig. 3 illustrates an example method for performing step 230 of method 200. In the example of Fig. 3, step 230 comprises a sub-step 231 of generating, for at least one value of at least one of the one or more sleep parameters in the titration data, a comparison result between said value and a corresponding value in the conventional sleep data, to thereby produce one or more comparison results.

Step 230 may further comprise a sub-step 232 of determining whether the one or more comparison results satisfy the first predetermined condition. In response to a determination that the one or more comparison results do not satisfy the first predetermined condition, sub-steps 231 and 232 may be repeated at a predetermined interval.

In response to a determination that the one or more comparison results satisfy the first predetermined condition, step 230 may proceed to sub-step 233 of processing the sleep influencing factor information in the titration data and the sleep influencing factor information in the conventional sleep data to determine, if present, the one or more differences between the PAP titration sleep session and the at least one conventional sleep session.

Optionally, where the sleep influencing factor information in the conventional sleep data comprises a subjective user assessment of the subject's sleep experience and information relating to one or more other sleep influencing factors, sub-step 233 may itself comprise a sub-step 234 of processing the subjective user assessment of the subject's sleep experience to determine whether or not a second predetermined condition is satisfied.

In response to a determination that the second predetermined condition is not satisfied, sub-steps 231, 232 and 234 may be repeated at a predetermined interval.

In response to a determination that the second predetermined condition is satisfied, sub-step 233 may proceed to sub-step 235, in which the information relating to one or more other sleep influencing factors in the titration data and in the conventional sleep data is processed to determine, if present, the one or more differences between the PAP titration sleep session and the at least one conventional sleep session.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (130) for generating a predictive indicator representing a difference between a PAP titration sleep session and at least one conventional sleep session of a subject (110), the processing system being configured to:
obtain titration data (165) for the subject, wherein the titration data was acquired during the PAP titration sleep session and comprises at least one value for each of one or more sleep parameters and sleep influencing factor information;
obtain conventional sleep data for the subject, wherein the conventional sleep data was acquired during the at least one conventional sleep session in which positive airway pressure, PAP, therapy was delivered by a PAP device (120), and wherein the conventional sleep data comprises at least one value for each of the one or more sleep parameters (145) and sleep influencing factor information (155) relating to one or more sleep influencing factors;
process the titration data and the conventional sleep data to determine, if present, one or more differences between the PAP titration sleep session and the conventional sleep session; and
generate a predictive indicator that indicates whether or not, if present, the one or more differences between the PAP titration sleep session and the at least one conventional sleep session satisfy a first predetermined condition.

2. The processing system (130) of claim 1, wherein the step of processing the titration data (165) and the conventional sleep data (145, 155) to determine, if present, the one or more differences between the PAP titration sleep session and the at least one conventional sleep session comprises:
generating, for at least one value of at least one of the one or more sleep parameters in the titration data, a comparison result between said value and a corresponding value in the conventional sleep data, to thereby produce one or more comparison results; and
in response to the one or more comparison results satisfying the first predetermined condition, processing the sleep influencing factor information in the titration data and the sleep influencing factor information in the conventional sleep data to determine, if present, one or more differences between sleep influencing factor information in the PAP titration sleep session and the at least one conventional sleep session.

3. The processing system (130) of claim 2, wherein:
the sleep influencing factor information (155) in the conventional sleep data comprises a subjective user assessment of the subject's sleep experience and information relating to one or more other sleep influencing factors;
the sleep influencing factor information in the titration data (165) comprises information relating to the one or more other sleep influencing factors; and
the step of processing the sleep influencing factor information to determine, if present, the one or more differences comprises:
processing the subjective user assessment of the subject's sleep experience to determine whether or not a second predetermined condition is satisfied; and
in response to the second predetermined condition being satisfied, processing the information relating to the one or more other sleep influencing factors in the titration data and the information relating to the one or more other sleep influencing factors in the conventional sleep data to determine the one or more differences between the PAP titration sleep session and the at least one conventional sleep session.

4. The processing system (130) of any of claims 1 to 3, wherein the processing system is further configured to control a first output user interface (170) to provide a first user-perceptible output of the one or more differences.

5. The processing system (130) of any of claims 1 to 4, wherein the processing system is further configured to process the one or more differences between the PAP titration sleep session and the at least one conventional sleep session to determine a recommended action.

6. The processing system (130) of claim 5, wherein the processing system is further configured to control a second output user interface to provide a second user-perceptible output of the recommended action.

7. The processing system (130) of claim 5 or 6, wherein:
the recommended action comprises an adjusted pressure to be provided by the PAP device (120); and
the processing system is further configured to generate a control signal configured to control the PAP device to provide air to the subject at the adjusted pressure.

8. The processing system (130) of any of claims 5 to 7, wherein the step of determining a recommended action comprises:
processing the one or more differences between the PAP titration sleep session and the at least one conventional sleep session to determine an initial recommended action;
processing the titration data (165) and the conventional sleep data (145, 155) to identify, for each of one or more aspects of the subject's behavior and/or environment, a relative effect of the aspect on a sleep quality for the subject; and
processing the relative effect of each aspect and the initial recommended action to determine the recommended action.

9. The processing system (130) of any of claims 1 to 8, wherein the processing system is further configured to:
process the at least one value for at least one of the one or more sleep parameters in the titration data (165) and/or the conventional sleep data (145) to detect an arousal of the subject;
process the titration data and/or the conventional sleep data to determine whether or not the detected arousal is caused by a sleep disordered breathing, SDB, event; and
in response to a determination that the detected arousal is caused by an SDB event, determine an adjustment to be made to the PAP therapy provided to the subject.

10. The processing system (130) of claim 9, wherein, in response to a determination that the detected arousal is not caused by an SDB event, the processing system is further configured to:
process the titration data (165) and/or the conventional sleep data (145, 155) to determine a cause of the detected arousal; and
control a third output user interface to provide a third user-perceptible output of the cause of the detected arousal.

11. The processing system (130) of any of claims 1 to 10, wherein:
the at least one value for each of the one or more sleep parameters (145) in the conventional sleep data is acquired by one or more sleep parameter sensors (140) comprising at least one of: an airflow sensor, a pressure sensor, a cardiorespiratory sensor, a brain activity sensor, a muscle sensor, and/or a pulse oximetry sensor; and
the sleep influencing factor information (155) in the conventional sleep data is acquired by one or more sleep influencing factor sensors (150) comprising at least one of: a user input device, an accelerometer, a particle sensor and/or an exhaled breath sensor.

12. The processing system (130) of any of claims 1 to 11, wherein the conventional sleep data (145, 155) comprises data acquired during a plurality of conventional sleep sessions.

13. A positive airway pressure, PAP, device (120) comprising:
one or more sleep parameter sensors (140) configured to acquire at least one value for each of one or more sleep parameters;
one or more sleep influencing factor sensors (150) configured to acquire sleep influencing factor information; and
the processing system (130) of any of claims 1 to 12.

14. A computer-implemented method (200) for generating a predictive indicator representing a difference between a PAP titration sleep session and at least one conventional sleep session of a subject (110), the computer-implemented method comprising:
obtaining titration data (165) for the subject, wherein the titration data was acquired during the PAP titration sleep session and comprises at least one value for each of one or more sleep parameters and sleep influencing factor information relating to one or more sleep influencing factors;
obtaining conventional sleep data for the subject, wherein the conventional sleep data was acquired during the at least one conventional sleep session in which positive airway pressure, PAP, therapy was delivered by a PAP device (120), and wherein the conventional sleep data comprises at least one value for each of the one or more sleep parameters (145) and sleep influencing factor information (155) relating to one or more sleep influencing factors;
processing the titration data and the conventional sleep data to determine, if present, one or more differences between the PAP titration sleep session and the at least one conventional sleep session; and
generating a predictive indicator that indicates whether or not, if present, the one or more differences between the PAP titration sleep session and the at least one conventional sleep session satisfy a first predetermined condition.

15. A computer program product comprising computer program code means which, when executed on a computing device having a processing system, cause the processing system to perform all of the steps of the method (200) according to claim 14.
